# EUROPEAN PATENT SPECIFICATION

(11) **EP 0 784 045 B1**
(45) Date of publication and mention of the grant of the patent: **23.08.2000**
(21) Application number: 96300280.3
(22) Date of filing: 13.01.1996
(51) Int. Cl.: C07C 55/14, C07C 51/31, B01J 31/18

(54) **A process for the preparation of adipic acid**
Verfahren zur Herstellung von Adipinsäure
Procédé de préparation d'acide adipique

(43) Date of publication of application: 16.07.1997
(73) Proprietor: Council of Scientific and Industrial Research, New Delhi 110 001 (IN)
(72) Inventor: Ratnasamy, Paul, Pune 411 008, Maharashtra (IN); Raja, Robert, Pune 411 008, Maharashtra (IN)
(74) Representative: Low, Peter John

(56) References cited:
- EP-A- 0 471 561
- EP-A- 0 532 326
- EP-A- 0 532 327
- WO-A-91/01806
- DE-A- 3 930 145

## Description

This invention relates to a process for the preparation of adipic acid. More particularly, the present invention relates to a process for the preparation of adipic acid by the oxidation of cyclohexane, using molecular oxygen as the oxidant and a solid organotransition metal complex as a catalyst.

### BACKGROUND OF THE INVENTION

Adipic acid is a major commodity chemical used as an intermediate in the manufacture of nylon 6,6, urethane foams, acidulant in baking powder and lubricating additives. The great majority of adipic acid on the market is made from cyclohexane, generally via KA oil which is a mixture of cyclohexanol and cyclohexanone. Adipic acid is made by a two step process from cyclohexane. In the first step cyclohexane is oxidised at a temperature range 150 to 175°C and a pressure of 115 to 175 psi in the presence of a soluble catalyst like cobalt napthenate or octoate in a concentration of 0.3 to 3 ppm. Conversions are usually in the range of 3 to 8% with selectivities in the range of 70 to 80%. In the second step, the mixture of cyclohexanol and cyclohexanone, which are formed by the oxidation of cyclohexane in the first step, are oxidised by nitric acid to adipic acid. Numerous byproducts are formed. The byproducts include formic acid, butyric acid, valeric acid, caproic acid, etc. In addition gaseous byproducts like carbon monoxide and dioxide are also formed.

There are many drawbacks in the two-step process for the oxidation of cyclohexane to adipic acid mentioned hereinabove and in commercial practice worldwide extensively. One drawback is the low level (3-5 %)of cyclohexane conversion necessitating the large recycle (more than 95%) of unreacted cyclohexane incurring thereby an expenditure of a large amount of process energy. A second major disadvantage of such processes is the use of nitric acid in the oxidation of KA oil to adipic acid. Large amounts (mole equivalent of nitric acid used) of nitrogen oxide vapours are released in the process which constitute an environmental hazard. Yet another drawback of the two step prior art process is the large amount of liquid and gaseous byproducts formed in both steps of the process leading to severe problems in their disposal. Eventhough many of these processes are practised commercially, all of them suffer from high cost due to both such multi step operations and the use of nitric acid as well as from pollution problems caused by the discharge of ozone depleting nitrogen oxide byproducts mentioned hereinabove.

### PRIOR ART REFERENCES

Other process options for the manufacture of adipic acid without the use of nitric acid have been proposed as for example in US patent No.3,390174 and British patent No.1,304,855. However, the air oxidation processes proposed in these patents are multi step processes with poor selectivity (in the range 30-50%) and require difficult high cost adipic acid recovery processes. An additional problem in all the prior art processes using molecular oxygen or air as oxidant and soluble homogeneous catalysts is the necessity to recover or dispose off the soluble metal catalysts that are used in such processes. Hence an air oxidation process that provides good yields of adipic acids free of significant by-products, such as succinic, glutaric and caproic acids and using a solid oxidation catalyst will be highly desirable. There have been many references in the prior art, to the one step molecular oxygen oxidation of cyclohexane to adipic acid. Japanese Patent No.45-16444 claims the oxidation of cyclohexane in acetic acid using cobalt acetate and acetaldehyde as catalysts at 80°C, oxygen at 225 psi, giving a conversion of 96% and a selectivity to adipic acid of 70%. British Patent 1,143,213 claims the oxidation of cyclohexane at 114 to 119°C, 250 psi in acetic and propionic acid using manganese stearate as catalyst. US Patent 4,263,453 claims oxidation of cyclohexane at 95°C, 300 psi in acetic acid containing a little water and using cobalt acetate as a catalyst giving a conversion of 92% and a selectivity to adipic acid of 80%. Until now however the seemingly attractive direct oxidation routes using molecular oxygen have not proven to be commercially and environmentally viable because of the soluble metal catalysts, such as cobalt acetate and cobalt napthenate used therein, as well as the low conversion (3-5%) and selectivity (30-50%) obtained in such processes. A review of the known single stage oxidation processes using homogeneous catalysts for the preparation of adipic acid from cyclohexane are discussed by K. Tanaka et al in the journals Chemtech, 555-559 (1974) and Hydrocarbon Processing, 53,114-120 (1974). Additional references for the single step direct oxidation of cyclohexane to adipic acid using soluble homogeneous catalysts include US patents 3,231,608; 2,589,648; 4,032,569; 4,263,453; 4,158,739; 5,321,157; as well as the article by G.N. Kulsrestha et al in Chem.Tech.Biotechnol., 50, 57-65 (1991).

The use of solid catalyst in the oxidation of cyclohexane to adipic acid is known in the prior art. F.T. Starzyk et al reported in the journal Studies in Surface Science and Catalysis Vol.84, pages 1419-1424 (1994) that using tertiary butyl hydroperoxide, but not molecular oxygen, as the source of oxygen and iron phthalocyanine encapsulated in Y zeolite as the catalyst, cyclohexane could be oxidised to adipic acid. One significant drawback of the process was the very slow rates of oxidation of cyclohexane thereby rendering the process commercially not attractive. Fig.2 of the article of Starzyk et al mentioned hereinabove, for example, teaches that 300 hours of reaction time are needed to achieve a cyclohexane conversion of about 35% at 60°C. Moreover, significant quantities of adipic acid started appearing in the liquid product only after about 600 hours, the major products being cyclohexanone and hydroxy ketone upto this time. Kraushaar et al in European Patent 519,569 (1992) and Lin, S.S. and Weng, H.S. in the Journal of Applied Catalysis, Vol.A (105) page 229 (1993) have claimed the use of a cobalt-substituted aluminophosphate-5 as a heterogeneous catalyst for the autooxidation of cyclohexane in acetic acid as solvent. The intermediate cyclohexanol is converted to the more stable cyclohexylacetate. Hence, this system suffers from the inherent disadvantages of requiring acetic acid solvent and separate hydrolysis and dehydrogenation steps. R.A. Sheldon et al have recently claimed in International Patent PCT/NL 94/6319 (1994) and in the article in Journal of Catalysis, Vol.153, pages 1-8 (1995) that chromium substituted aluminophosphate-5 is a heterogeneous catalyst for the oxidation of cyclohexane at 115-130°C, 75 psi O₂ and 300 psi air in the presence of a small amount of an alkyl hydroperoxide initiator to yield cyclohexanone as the major product. Cyclohexanol conversion levels were in the range, 3-10% wt. Cyclohexanone and cyclohexanol, the former in predominant proportions, were the main products. Significant quantities of byproducts, mainly dibasic acids like succinic, glutaric and adipic acids were also produced due to the high temperatures of the reaction.

It is thus evident that there is a need for the development of a process for the oxidation of cyclohexane to adipic acid in significant yields (at least 10-15% wt, for example) and using solid, recyclable catalysts and operating at a low enough temperature (below 100° C, for example) to avoid the production of undesirable byproducts like succinic, glutaric, caproic and hydroxy caproic acids.

### OBJECTS OF THE INVENTION

It is, therefore, an object of the present invention to provide a process for the preparation of adipic acid by the oxidation of cyclohexane using a catalyst which would remain in the solid state at the end of the oxidation reaction thereby facilitating the easy separation, recovery and recycle of the catalyst from the reaction products without having any adverse impact on the environment.

Another object of the present invention is to provide an improved process whereby the yield of adipic acid would be higher, in the range of 10 to 25 %, than in the prior art processes. Yet another objective of the present invention is to provide an improved process for the preparation of adipic acid at a temperature below 100^{°}C wherein a large number of byproducts due to thermal oxidation of cyclohexane, cyclohexanol, cyclohexanone and adipic acid reactions are generated.

### DETAILED DESCRIPTION OF THE INVENTION

Pthalocyanines consist of large, planar, conjugated, ring systems which serve as tetradentate ligands. Metallic cations can be easily accommodated at the center of these systems with the four nitrogens as the ligating atoms. Metal containing pthalocyanine compounds are useful as chemical reagents of a catalytic nature, more particularly in directing certain oxidative processes. Many known pthalocyanines have been judged to suffer certain drawbacks by being deficient in the combination of properties desired for many candidate uses, such as in the oxidation of alkanes and more particularly in the oxidation of cyclohexane. One major drawback of homogeneous pthalocyanine catalysts in industrial oxidation processes is the formation of aggregates in solution which significantly deactivates these catalysts.

Due to our continued research in this area we observed that the organotransition metal complexes used as catalysts are solids insoluble in cyclohexane or the reaction products arising from oxidation of cyclohexane. Hence they do not undergo aggregation or change of phase during the oxidation wherein such changes are known to lead to catalyst deactivation problems.

Another drawback of pthalocyanines used in the prior art as catalysts for alkane oxidation is their low oxidative stability which is due to the easy oxidisability of the hydrogen atoms attached to the nucleus of the pthalocyanines.

We have found that the oxidative stability as well as the catalytic activity of the metal pthalocyanines used as catalysts in the oxidation of cyclohexane are enhanced by replacing the hydrogens from the pthalocyanines by electron withdrawing groups like the halogens, nitro or cyano groups thereby rendering the metal ions easier to reduce leading to an improved oxidation activity and stability of the catalysts during the reaction.

There are a total of 16 hydrogen atom positions on such pthalocyanine molecules which can in principle, be substituted by other substituents. We have observed that when some or all of the hydrogen atoms of the said pthalocyanines are substituted by one or more electron withdrawing groups such as halogen, nitro or cyano groups or mixtures of such groups there is substantial improvement in selectivity and conversion to adipic acid.

Accordingly, the present invention provides an improved process for the oxidation of cyclohexane to adipic acid which comprises reacting cyclohexane with molecular oxygen in the presence of a solid catalyst consisting of an organotransition metal complex wherein some or all of the hydrogen atoms of the said organotransition metal complex have been substituted by one or more electron withdrawing groups, at a temperature in the range of 20°C to 80°C, at a pressure in the range of 345 to 69 000 kPa (5 to 1000 psi) pressure, in the presence or absence of solvents, with or without a promoter and isolating the adipic acid formed by conventional methods, such as filtration and centrifugation. The organotransition metal complex containing pthalocyanines and porphyrins is used as catalyst. The transition metal is selected from iron, cobalt, copper, chromium , manganese or mixtures thereof.

Some nonlimiting examples of such organo transition metal complexes used as catalysts in the oxidation of cyclohexane to adipic acid are iron halopthalocyanines, copper halo pthalocyanines, cobalt halo pthalocyanines, chromium halo pthalocyanines, manganese halo pthalocyanines, iron nitro pthalocyanines, copper nitro pthalocyanines, chromium nitro pthalocyanines, cobalt nitro pthalocyanines, manganese nitro pthalocyanines, manganese cyano pthalocyanines, copper cyano pthalocyanines and chromium cyano pthalocyanines, iron halo porphyrins, copper tetra halo porphyrins, manganese halo porphyrins, cobalt halo porphyrins, manganese halo tetra phenyl porphyrins, copper halo tetra phenyl porphyrins and cobalt halo tetra phenyl porphyrins.

The electron withdrawing groups attached to the organotransition metal complex is selected from the halogens, fluorine, chlorine, bromine or iodine or the nitro or cyano groups.

In a preferred embodiment of the present invention, the oxidation of cyclohexane by molecular oxygen is catalysed by the halogen, cyano or nitro pthalocyanines of the metals iron, cobalt, copper, chromium or manganese.

In yet another embodiment of the present invention, the source of molecular oxygen can be pure oxygen gas, air or a mixture of oxygen and an inert gas diluent like nitrogen.

In yet another embodiment of the present invention, the above mentioned oxidation reaction can be carried out in the presence or absence of solvents. It may be an advantageous option to carry out the said oxidation reaction in the presence of a suitable solvent which would maintain the oxidation products like adipic acid in the dissolved state during the course of the reaction, thereby facilitating the separation of the said adipic acid from the solid catalysts. Suitable solvents for such use include acetonitrile, methanol, water, butanol and cyclohexanol. Examples of such solvents which can be used in the process of the present invention include acetonitrile, acetone, benzene or any other organic solvent which is inert under the oxidation reaction conditions.

In another embodiment of the present invention, the rates of the oxidation of cyclohexane to adipic acid may be significantly enhanced by addition of very small catalytic quantities of a promoter. Examples of such promoters include alkyl hydroperoxide and dialkylperoxides. Cyclohexyl hydroperoxide, cumyl peroxide, tertiary butyl hydroperoxide are some of the examples of such promoters which may be present in concentrations not exceeding 1% by weight of cyclohexane and more preferably 0.1% by weight of cyclohexane.

In yet another embodiment of the present invention, the organotransition metal complex may be encapsulated in a solid matrix. Due to the greater dispersion of the organotransition metal complex catalyst in solid matrices and the consequent enhanced stability of the structural integrity of the catalyst significant process advantages like greater activity, stability and easy recovery and recyclability of the catalyst are observed. Examples of such solid matrices include inorganic oxide like silica, alumina, molecular sieves, zeolites as well as organic polymeric materials, like polystyrene.

In a still another embodiment of the process of the present invention that due to the high activity the catalysts used herein, the oxidation reaction can be carried out at temperatures much below those used in the prior art and preferably below 80^{°}C, thereby leading to much lower yields of undesired side products like succinic, glutaric and caproic acids.

In the process of the present invention, the oxidation of cyclohexane to adipic acid proceeds via the intermediates cyclohexanol and cyclohexanone. Hence, if the oxidation to adipic acid is incomplete the product mixture after the reaction may contain significant quantities of cyclohexanol and cyclohexanone. The cyclohexanone and cyclohexanol obtained as intermediates in the oxidation of cyclohexane to adipic acid may either be recycled back to the cyclohexane oxidation zone or be converted to other valuable products, like nylon-6.

The details of the present invention is described in the examples given below which are provided by way of illustration only and therefore should not be construed to limit the scope of the invention.

### Example-1

In an autoclave, 5 g of cyclohexane and 0.3 g of solid iron tetra deca bromo pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The conversion of cyclohexane was 8% wt and the yield of adipic acid was 4% wt.

### Example-2

In an autoclave, 5 g of cyclohexane and 0.3 g of solid cobalt tetra deca chloro pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The results are given in Table 1.

### Example-3

In an autoclave, 5 g of cyclohexane and 0.3 g of solid copper tetra deca chloro pthalocyanine were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The results are given in Table 1.

### Example-4

In an autoclave, 5 g of cyclohexane and 0.3 g of solid chromium tetra deca fluoro pthalocyanine were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The results are given in Table 1.

### Example-5

In an autoclave, 5 g of cyclohexane and 0.3 g of solid manganese tetra deca fluoro pthalocyanine were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The results are given in Table 1.

Table 1 indicates the wt % conversion of cyclohexane, wt % yield of adipic acid and the wt % yield of cyclohexanol plus cyclohexanone when using different organotransition metal complexes as catalysts and using the conditions mentioned herein above (Examples 2-5).

**Table 1**

| | Ex-2 | Ex-3 | Ex-4 | Ex-5 |
|---|---|---|---|---|
| Pthalocyanine | Cobalt | Copper | Chromium | Manganese |
| | | | | |
| Conv. cyclohexane % wt | 6 | 7 | 8 | 12 |
| | | | | |
| Yield of adipic acid, % wt | 3 | 4 | 4 | 7 |
| | | | | |
| Yield of % wt cyclohexanone plus cyclohexanol | 3 | 3 | 4 | 5 |

### Example-6

In an autoclave, 5 g of cyclohexane and 0.3 g of solid iron deca nitro pthalocyanine were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The results are given in Table 2.

### Example-7

In an autoclave, 5 g of cyclohexane and 0.3 g of solid cobalt deca nitro pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The results are given in Table 2.

### Example-8

In an autoclave, 5 g of cyclohexane and 0.3 g of solid copper deca nitro pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The results are given in Table 2.

### Example-9

In an autoclave, 5 g of cyclohexane and 0.3 g of solid chromium deca nitro pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The results are given in Table 2.

### Example-10

In an autoclave, 5 g of cyclohexane and 0.3 g of solid manganese deca nitro pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The results are given in Table 2.

Table 2 indicates the wt % conversion of cyclohexane, wt % yield of adipic acid and the wt % yield of cyclohexanol plus cyclohexanone when using different organotransition metal complexes as catalysts and using the conditions mentioned herein above (Examples 6-10)

**Table - 2**

| | Ex-6 | Ex-7 | Ex-8 | Ex-9 | Ex-10 |
|---|---|---|---|---|---|
| Pthalocyanine | Fe | Co | Cu | Cr | Mn |
| | | | | | |
| Cyclohexane conv, % wt | 9 | 13 | 15 | 12 | 15 |
| | | | | | |
| Adipic acid yield % wt | 4 | 5 | 10 | 6 | 11 |
| | | | | | |
| Yield of % wt cyclohexanone plus cyclohexanol | 5 | 8 | 5 | 6 | 4 |

### Example-11

In an autoclave, 5 g of cyclohexane and 0.3 g of solid iron tricyano pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid was 3 % wt.

### Example-12

In an autoclave, 5 g of cyclohexane and 0.3 g of solid cobalt tricyano pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid was 6 % wt.

### Example-13

In an autoclave, 5 g of cyclohexane and 0.3 g of solid copper tricyano pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid was 8 % wt.

### Example-14

In an autoclave, 5 g of cyclohexane and 0.3 g of solid chromium cyano pthalocyanine were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid was 9 % wt.

### Example-15

In an autoclave, 5 g of cyclohexane and 0.3 g of solid manganese cyano pthalocyanine were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid was 9 % wt.

### Example-16

In an autoclave, 5 g of cyclohexane, 0.3 g of solid iron tetra deca bromo pthalocyanine, 5 g of acetonitrile solvent, and 0.08 g of tert. butyl hydroperoxide promoter were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The conversion of cyclohexane was 20% and the yield of adipic acid was 12% wt.

### Example-17

In an autoclave, 5 g of cyclohexane, 0.3 g of solid iron tetra deca chloro pthalocyanine, 5 g of methanol solvent, and 0.08 g of tert. butyl hydroperoxide promoter were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The conversion of cyclohexane was 22% and the yield of adipic acid was 15% wt.

### Example-18

In an autoclave, 5 g of cyclohexane, 0.3 g of solid copper tetra deca chloro pthalocyanine, 5 g of methanol solvent, and 0.08 g of ditert. butyl peroxide promoter were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The conversion of cyclohexane was 24% and the yield of adipic acid was 15% wt.

### Example-19

In an autoclave, 5 g of cyclohexane, 0.3 g of solid copper tetra deca bromo pthalocyanine encapsulated in the aluminosilicate molecular sieve-Y, 5 g of methanol solvent, and 0.08 g of ditert. butyl peroxide promoter were stirred at 50^{°}C with a continous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds.The conversion of cyclohexane was 22% and the yield of adipic acid was 16% wt.

### Example-20

In an autoclave, 5 g of cyclohexane, 0.3 g of solid copper tetra deca chloro pthalocyanine encapsulated in polystyrene, 5 g of methanol solvent, and 0.08 g of ditert. butyl peroxide promoter were stirred at 50^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The conversion of cyclohexane was 18% and the yield of adipic acid was 14% wt.

### Example-21

In an autoclave, 2.5 g of cyclohexane, 1.25 g of cyclohexanol, 1.25 g of cyclohexanone, 0.3 g of solid iron tetra deca bromo pthalocyanine, 5 g of acetonitrile solvent, and 0.08 g of tert. butyl hydroperoxide promoter were stirred at 50°C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The yield of adipic acid on the basis of total cyclohexane plus cyclohexanone plus cyclohexanol was 30% wt, compared to 12% wt when cyclohexane was used as the only substrate for oxidation.

### Example-22

In an autoclave, 5 g of cyclohexane and 0.5 g of solid iron tetra chloro porphyrin were stirred at 60^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The conversion of cyclohexane was 6 % and the yield of adipic acid was 2.5 % by wt.

### Example-23

In an autoclave, 5 g of cyclohexane, 0.5 g of solid manganese hexachloro tetraphenyl porphyrin, 0.1 g of tertiary butyl hydroperoxide and 5 g of acetonitrile were stirred at 60^{°}C with a continuous bubbling of air for 8 hrs. At the end of the reaction, 10 ml of methanol was added to the products (unreacted cyclohexane, cyclohexanol, cyclohexanone and adipic acid) which were then separated from the solid catalyst by centrifugation and analysed by gas chromatography (Shimadzu GC R1A) using a carbowax column and flame ionization detector (FID). The identity of the products was confirmed by GC mass spectroscopy (Shimadzu GCMS-QP 2000A) using standard compounds. The conversion of cyclohexane was 16 % and the yield of adipic acid was 7 % by wt.

## Claims

1. A process for the oxidation of cyclohexane to adipic acid, which comprises reacting cyclohexane with molecular oxygen in the presence of a solid catalyst containing an organotransition metal complex wherein some or all of the hydrogen atoms of the said organotransition metal complex have been substituted by one or more electron withdrawing groups, wherein the organotransition metal complex contains pthalocyanines or porphyrins, wherein the transition metal is selected from iron, cobalt, copper, chromium, manganese or mixtures thereof, wherein the said electron withdrawing group is selected from halogens, a nitro group, a cyano group or mixtures thereof, wherein the process is performed at a temperature in the range of 20°C to 80°C, at a pressure in the range of 345 to 69 000 kPa (5 to 1000 psi) in the presence or absence of solvents, with or without promoter and isolating the adipic acid formed by conventional methods such as filtration and centrifugation.

2. A process according to claim 1 wherein the source of molecular oxygen is oxygen, air or a mixture of oxygen and an inert gas like nitrogen.

3. A process according to claim 1 wherein the oxidation reaction is carried out in the presence of solvents selected from acetonitrile, methanol, butanol or cyclohexanol.

4. A process according to claim 1 wherein a promoter selected from alkyl hydroperoxide, dialkyl peroxide or mixtures thereof is used in the reaction.

5. A process according to claim 1 wherein the concentration of the promoter in the reaction mixture does not exceed 1 % by weight of the cyclohexane.

6. A process according to claim 1 wherein the organotransition metal complex is encapsulated in a solid matrix.

7. A process according to claim 6 wherein the solid matrix used is an inorganic oxide selected from silica, alumina, aluminosilicates or molecular sieves in the form of zeolites.

8. A process according to claim 6 wherein the solid matrix is an organic polymer in the form of polystyrene.

9. A process according to claim 6 wherein the solid matrix contains both an inorganic oxide in the form of silica and an organic polymer in the form of polystyrene.

10. A process according to claim 1 wherein the cyclohexane feed is admixed with cyclohexanone and cyclohexanol.

## Patentansprüche

1. Verfahren zur Oxidation von Cyclohexan zu Adipinsäure, das umfasst, dass Cyclohexan mit molekularem Sauerstoff in Gegenwart eines festen Katalysators, der einen Organoübergangsmetallkomplex enthält, umgesetzt wird, wobei einige oder alle Wasserstoffatome des Organoübergangsmetallkomplexes durch eine oder mehrere elektronenanziehende Gruppen ersetzt wurden, wobei der Organoübergangsmetallkomplex Phthalocyanine oder Porphyrine enthält, wobei das Übergangsmetall ausgewählt ist aus Eisen, Cobalt, Kupfer, Chrom, Mangan oder Mischungen davon, wobei die elektronenanziehende Gruppe ausgewählt ist aus Halogenen, einer Nitrogruppe, einer Cyanogruppe oder Mischungen davon, wobei das Verfahren bei einer Temperatur im Bereich von 20 bis 80°C und bei einem Druck im Bereich von 34.5 bis 6900 kPa (5 bis 1000 psi) in Gegenwart oder Abwesenheit von Lösungsmitteln, mit oder ohne Promotor, durchgeführt wird und die gebildete Adipinsäure mit üblichen Methoden, z.B. durch Filtration und Zentrifugation, isoliert wird.

2. Verfahren nach Anspruch 1, wobei die Quelle für molekularen Sauerstoff Sauerstoff, Luft oder eine Mischung aus Sauerstoff und einem Inertgas, wie Stickstoff, ist.

3. Verfahren nach Anspruch 1, wobei die Oxidationsreaktion in Gegenwart von Lösungsmitteln durchgeführt wird, die ausgewählt sind aus Acetonitril, Methanol, Butanol oder Cyclohexanol.

4. Verfahren nach Anspruch 1, wobei ein Promotor, ausgewählt aus Alkylhydroperoxid, Dialkylperoxid oder Mischungen davon, für die Reaktion verwendet wird.

5. Verfahren nach Anspruch 1, wobei die Konzentration des Promotors in der Reaktionsmischung 1 Gew.-% bezogen auf Cyclohexan nicht übersteigt.

6. Verfahren nach Anspruch 1, wobei der Organoübergangsmetallkomplex in einer festen Matrix eingekapselt ist.

7. Verfahren nach Anspruch 6, wobei die verwendete feste Matrix ein anorganisches Oxid ist, das ausgewählt ist aus Siliciumdioxid, Aluminiumoxid, Aluminosilicaten oder Molekularsieben in Form von Zeolithen.

8. Verfahren nach Anspruch 6, wobei die feste Matrix ein organisches Polymer in Form von Polystyrol ist.

9. Verfahren nach Anspruch 6, wobei die feste Matrix sowohl ein anorganisches Oxid in Form von Siliciumdioxid als auch ein organisches Polymer in Form von Polystyrol enthält.

10. Verfahren nach Anspruch 1, wobei die Cyclohexanbeschikkung mit Cyclohexanon und Cyclohexanol vermischt wird.

## Revendications

1. Procédé pour l'oxydation du cyclohexane en acide adipique qui comprend
- la réaction du cyclohexane avec de l'oxygène moléculaire en présence d'un catalyseur solide contenant un complexe organométallique de transition caractérisé en ce que les atomes d'hydrogène du complexe organométallique de transition ont été substitués en totalité ou en partie par un ou plusieurs groupements électroattracteurs, le complexe organométallique de transition contient des pthalocyanines ou porphyrines, le métal de transition est choisi parmi le fer, le cobalt, le cuivre, le chrome, le manganèse ou leurs mélanges, le groupement électroattracteur est choisi parmi les halogènes, le groupement nitro, le groupement cyano ou leurs mélanges, avec ledit procédé étant réalisé à une température comprise entre 20°C et 80°C, à une pression comprise entre 34.5 à 6900 kPa. (5 à 1000 psi) en présence ou en absence de solvants, avec ou sans activateur et
- l'isolement de l'acide adipique formé à l'aide de techniques conventionnelles telles que filtration et centrifugation.

2. Procédé selon la revendication 1, caractérisé en ce que la source en oxygène moléculaire est l'oxygène, l'air ou un mélange de l'oxygène avec un gaz inerte tel l'azote.

3. Procédé selon la revendication 1, caractérisé en ce que la réaction d'oxydation est réalisée en présence de solvants choisis parmi l'acétonitrile, le méthanol, le butanol ou le cyclohexanol.

4. Procédé selon la revendication 1, caractérisé en ce que l'on utilise dans la réaction un activateur choisi parmi les hydroperoxyde d'alkyle, peroxyde dialkyle ou leurs mélanges.

5. Procédé selon la revendication 1, caractérisé en ce que la concentration en activateur dans le milieu réactionnel n'excède pas 1% en poids du cyclohexane.

6. Procédé selon la revendication 1, caractérisé en ce que le complexe organométallique de transition est encapsulé dans une matrice solide.

7. Procédé selon la revendication 6, caractérisé en ce que la matrice solide utilisée est un oxyde inorganique choisi parmi la silice, l'alumine, les aluminosilicates ou des tamis moléculaires de type zéolites.

8. Procédé selon la revendication 6, caractérisé en ce que la matrice solide est un polymère organique de type polystyrène.

9. Procédé selon la revendication 6, caractérisé en ce que la matrice solide contient à la fois un oxyde inorganique de type silice et un polymère organique de type polystyrène.

10. Procédé selon la revendication 1, caractérisé en ce que l'alimentation en cyclohexane est effectuée en mélange avec le cyclohexanone et le cyclohexanol.
